# EUROPÄISCHE PATENTANMELDUNG

(11) **EP 1 579 840 A1**
(43) Veröffentlichungstag der Anmeldung: **28.09.2005**
(21) Anmeldenummer: 05005077.2
(22) Anmeldetag: 09.03.2005
(51) Int. Cl.: A61K 6/00, A61K 6/027, A61K 6/06, A61C 13/083, C04B 37/02, C23C 24/08

(54) **Dentalkeramikhaftvermittler**

(30) Priorität: 23.03.2004 DE 102004014604
(71) Anmelder: KLEMA Dentalprodukte Ges.m.b.H., 6812 Meiningen (AT)
(72) Erfinder: Kleboth, Klaus, Dr., 6812 Meiningen (AT); Heinzle, Oliver, 6812 Meiningen (AT); Rettig, Karsten, Dr., 6812 Meiningen (AT)
(74) Vertreter: Behrmann, Niels

(57) **Zusammenfassung**

Die Erfindung betrifft einen Dentalkeramik-Haftvermittler zum Verbinden eines insbesondere mittels einer Nichtedelmetalllegierung realisierten Metall-Basisgerüsts (2) mit einer aufzubringenden dentalkeramischen Schicht (3,4,5), wobei der Haftvermittler (1) aus einem zwischen 10 und 50 Gew.-%, insbesondere zwischen 30 und 45 % Titan- und/oder Zinndioxid, zwischen 3 und 30 Gew.-% Bortrioxid und eine Glaskomponente aufweisenden Haftvermittlermaterial hergestellt ist.

## Beschreibung

Die vorliegende Erfindung betrifft einen Dentalkeramik-Haftvermittler nach dem Oberbegriff des Hauptanspruchs.

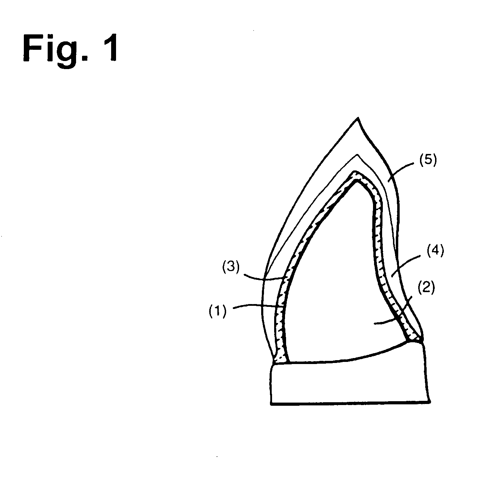

Derartige Haftvermittler sind aus dem Stand der Technik allgemein bekannt und dienen, wie exemplarisch in der Fig. 1 verdeutlicht wird, üblicherweise zum Herstellen einer besseren Haftung zwischen einem Metallgerüst 2 (etwa einer dentalen Brücke) und einer darauf aufzubringenden Keramikschicht 3; im dargestellten Ausführungsbeispiel sitzt auf der Haftvermittlerschicht 1 eine sog. Opakerschicht 3, welche wiederum eine Dentinschicht 4 sowie darauf eine Schneidemasse 5 trägt.

Dabei wird als Metallsubstrat für das Metallgerüst sowohl Edelmetall bzw. eine Edelmetalllegierung (typischerweise neben Gold auch Platin, Palladium, Silber) verwendet, alternativ eine Nicht-Edelmetalllegierung, vorzugsweise mit Chrom, Cobalt, Nickel, Molybdän, Wolfram, Vanadium, Niob, Silizium.

Dabei ist für eine dauerhafte Verbindung zwischen einer solchen Metalllegierung des Basisgerüsts und einer Dentalkeramik die physikalische und/oder chemische Haftung des Keramikmaterials am Metall entscheidend, und diese wiederum wird kritisch beeinflusst vom jeweiligen Wärmeausdehnungskoeffizienten (im weiteren: WAK). Mit anderen Worten, eine möglichst kompatible, weitgehend identisch verlaufende Wärmeausdehnung ist für eine dauerhafte, belastbare Verbindung wünschenswert.

Aus den zum Stand der Technik gehörenden Erkenntnissen über das Zustandekommen des Verbundes zwischen der dentalkeramischen Schicht und dem unterliegenden metallischen Basisgerüst ist bekannt, dass neben mechanischer Retention der Keramik an Oberflächenrauhigkeiten und Unterschnitten des Metalls durch eine geeignete Wahl der Wärmeausdehnungskoeffizienten von Keramik und Metall beim Abkühlungsprozess des keramischen Brennvorgangs die Keramik unter Druckspannung gesetzt wird. Dies führt in der Abkühlphase des keramischen Brennvorgangs zu einem Aufschrumpfen der Keramik auf dem Metallkörper und zur Kompression von Mikrorissen in der Keramik, mit der vorteilhaften Wirkung, dass die Keramik bei mechanischer Beanspruchung erst spät unter eine schädliche Zugbeanspruchung gerät. Auch wird die durch Oberflächenrauhigkeiten und Unterschnitte bedingte mechanische Haftung verbessert; mithin wird auch hierdurch die Haftfestigkeit der keramischen Beschichtung auf dem Metall-Basisgerüst erhöht.

Gleichwohl wird auch ein wesentlicher Beitrag zur wirksamen Metall-Keramik-Bindung chemischen Bindungskräften zugeschrieben.

Generell wird, um eine bessere Haftung zu erzielen, ein Haftvermittler (ein sog. Bonder) eingesetzt, welcher als dünne Schicht zwischen dem metallischen Substrat (Basisgerüst) und der Dentalkeramik aufgebracht bzw. aufgebrannt wird. Diese Bonder haben die Aufgabe, die chemische Haftung zu verbessern.

Aus dem Stand der Technik sind zahlreiche Haftvermittler bekannt, welche die Haftung zwischen Metallgerüst und Keramik verbessern sollen. So beschreibt etwa die DE 43 211 00 eine Keramik mit zugemischtem feinem Zinnpulver, welches als Reduktionsmittel die bei kupferhaltigen Metallgerüsten auftretenden Verfärbungen verhindert. Aus der DE 100 22 559 ist ferner ein Verbundwerkstoff bekannt, welcher neben einer keramischen Komponente als wichtigen Hauptbestandteil eine Metallpulvermischung aus Feingold und/oder einem refraktären Metalloxid enthält.

Die DE 30 46 334 beschreibt einen Haftvermittler, welcher neben einem Flussmittel Gold, Silber sowie eine hochschmelzende NE-Legierung enthält, und die DE 25 25 274 beschreibt schließlich einen Haftvermittler, welcher aus Goldpartikeln, einer Porzellanmasse, Zirkondioxid und Boroxiden besteht und als Suspension auf das Metallgerüst aufgetragen bzw. aufgebrannt wird.

All diesen bekannten Haftvermittlern ist jedoch gemeinsam, dass diese gerade im kritischen Übergangsbereich zwischen nicht-edelmetallhaltigen Basisgerüsten und der aufsitzenden Dentalkeramik oftmals schlechte Hafteigenschaften besitzen, nicht zuletzt aufgrund der unbefriedigend gelösten Differenzen im WAK der zu verbindenden Materialien.

Um die Wärmeausdehnungskoeffizienten von Metalllegierung und Keramik aufeinander abzustimmen, wurden zudem spezielle Metalllegierungen, sog. Aufbrennlegierungen und/oder -keramiken entwickelt, deren WAK aufeinander abgestimmt sind, so dass sie ein gleiches oder ähnliches Ausdehnungsverhalten zeigen. Damit werden die durch verschiedene Ausdehnung der Materialien auftretenden Belastungen (Risse, Sprünge oder Abplatzen der Keramik vom Metallgerüst) wirksam vermieden. Da jedoch prinzipiell für jede Metalllegierung eine spezielle, im WAK kompatible Keramik als Gegenstück vorhanden sein muss, ist eine derartige Lösung wenig praktikabel.

Da insbesondere für dentalkeramische Schichten auf edelmetallfreien Metall-Basisgerüsten der Wunsch besteht, die chemische Haftung zu verbessern, gleichzeitig jedoch Metalllegierungen und Keramiken mit unterschiedlichen Ausdehnungsverhalten miteinander zu kombinieren, besteht ein starkes Bedürfnis nach Haftvermittlern, welche in der Lage sind, im Hinblick auf ihr Ausdehnungsverhalten als mechanischer Puffer zwischen der (insbesondere edelmetallfreien) Oberfläche des Metall-Basisgerüsts und der aufzubringenden dentalkeramischen Schicht zu wirken.

Aufgabe der vorliegenden Erfindung ist es daher, einen verbesserten Haftvermittler zum Verbinden eines metallischen, insbesondere edelmetallfreien, Basisgerüsts mit einer dentalkeramischen Schicht zu schaffen, welcher eine hohe Haftwirkung erzeugt und geeignet ist, durch unterschiedliche thermische Spannungen erzeugte mechanische Beanspruchungen zwischen Basisgerüst und dentalkeramischer Schicht abzuschwächen.

Die Aufgabe wird durch den Dentalkeramik-Haftvermittler mit den Merkmalen des Hauptanspruchs, das Verfahren zum Herstellen einer Zahnprothese mit den Merkmalen des unabhängigen Verfahrensanspruchs 9 sowie die Verwendung nach dem Patentanspruch 7 gelöst. Vorteilhafte Weiterbildungen der Erfindung sind in den Unteransprüchen beschrieben.

In erfindungsgemäß vorteilhafter Weise ist der Dentalkeramik-Haftvermittler der Erfindung aus einem Haftvermittlermaterial (hier als Ausgangsprodukt zum Aufbringen auf das Basisgerüst vor einem Brennen zu verstehen) realisiert, welche neben den typisch glasbildenden Oxiden (im Rahmen der Erfindung als "Glaskomponente" bezeichnet) Bortrioxid im Bereich zwischen 3 und 30 Gew.-% sowie ein Metalldioxid in Form von Titan- und/oder Zinndioxid im Bereich zwischen 10 und 50 Gew.-% aufweist, wobei es sich hier als besonders bevorzugt herausgestellt hat, die Metalldioxidkomponente in einem Anteil zwischen 30 und 45 Gew.-% des Haftvermittler-materials bereitzustellen.

Erfindungsgemäß vorteilhaft hat es sich nämlich erwiesen, dass zwar offenbar das Bortrioxid allein nicht geeignet ist, als wirksamer Haftvermittler im vorliegenden Kontext zu wirken, in Verbindung mit den erfindungsgemäß herangezogenen Metalloxiden scheint sich jedoch unter dem Ausbilden von Oxidbrücken ein Netzwerk zu bilden, welches (da die Oberflächen gerade bei Nicht-Edelmetallen generell von einer festhaftenden Oxidschicht bedeckt sind) beim Aufbrennen zu einem festhaftenden Überzug führt. Wird, wie im Rahmen der vorliegenden Erfindung realisiert, das Bortrioxid zusammen mit einem Glas (oder als Bestandteil dieses Glases) eingesetzt, ist von einer mechanisch wirksamen Brückenfunktion als chemische Bindung zur Metalloberfläche auszugehen; wird dann noch, wie es üblicherweise einfach realisierbar ist, der WAK der erfindungsgemäß auf den Haftvermittler aufgebrachten dentalkeramischen Schicht mit darauf aufzubringenden Verblendkeramiken in weitgehende Übereinstimmung gebracht, steht eine äußerst feste Haftung der Gesamtanordnung zu erwarten.

Im Rahmen der Erfindung scheint dabei insbesondere der Zusatz von Metalldioxid, insbesondere des Titandioxid, in relativ hohen Anteilen als WAK-Puffer zu wirken, so dass im Rahmen der Erfindung die Kombination der drei Komponenten Bortrioxid, Metalldioxid und Glas einen universellen und äußerst leistungsfähigen Haftvermittler, insbesondere für die Verbindung von edelmetallfreien Legierungen mit Verblendkeramiken, ergibt.

In der praktischen Realisierung der Erfindung hat es sich dabei als besonders bevorzugt herausgestellt, Al₂O₃ als Bestandteil der Glaskomponente vorzusehen, sowie weiter bevorzugt K₂O und/oder Na₂O beizumischen. Auch ist es vorteilhaft, CaO als Bestandteil der Glaskomponente vorzusehen bzw. das erfindungsgemäß im Haftvermittlermaterial vorgesehene Bortrioxid zumindest teilweise auch als Bestandteil der Glaskomponente bereitzustellen.

Weiter vorteilhaft ist es, dem Haftvermittlermaterial Fe₂O₃ und/oder V₂O₅ zuzusetzen.

Im Ergebnis lässt sich so eine dentalkeramische Haftvermittlerschicht realisieren, welche im Hinblick auf ihre Haftwirkung überragende Eigenschaften aufweist: So wird etwa gemäß einschlägigen Vorschriften ein Haftwert von mindestens 25 MPa (gemessen etwa mittels der sog. Schwickerath'schen Rissbeginnprüfung gemäß EN ISO 9693) als ausreichend erachtet; regelmäßig zeigen dagegen die im Rahmen der vorliegenden Erfindung erzeugten Schichten Haftwerte in der Größenordnung von 80 MPa oder darüber.

Im Ergebnis hat daher die Erfindung gezeigt, dass das Einbringen vergleichsweise hoher Anteile an Bortrioxid und Metalloxid in Form von Titan- und/oder Zinndioxid in das Haftvermittlermaterial, zusammen mit der Glaskomponente, nicht nur zu hervorragender Haftung der Verblendkeramik am (bevorzugt edelmetallfreien) Metall führt, darüber hinaus ist die erfindungsgemäße Haftvermittlerschicht auch geeignet, Legierungen und Keramiken mit unterschiedlichen Wärmeausdehnungskoeffizienten haftfest und dauerhaft miteinander zu verbinden.

Weitere Vorteile, Merkmale und Einzelheiten der Erfindung ergeben sich aus der nachfolgenden Beschreibung bevorzugter Ausführungsbeispiele.

So verdeutlicht die Tabelle 1 eine bevorzugte Ausführungsform der Erfindung; sehr günstige Eigenschaften ergeben sich, wenn eingesetztes Titandioxid zwischen etwa 20 und 40 Gew.-% vorliegt, Zinndioxid zwischen etwa 10 und 30 Gew.-%. Die letzten vier Komponenten gelten im Rahmen der vorliegenden Erfindung als Bestandteile einer Glaskomponente.

**Tabelle 1**

| **Komponente** | **Gew.-%** |
|---|---|
| B₂O₃ | 2-30 |
| MeO₂ (Me = Ti, Sn) | 10-50 |
| SiO₂ | 10-55 |
| Al₂O₃ | 1-10 |
| (Na/K)₂ O | 1-20 |
| CaO | 0-4 |

Eine Variante der Erfindung zeigt die Tabelle 2, wobei auf Nicht-Edelmetalllegierungen eine nochmals erhöhte Haftkraft nachgewiesen werden konnte.

**Tabelle 2**

| **Komponente** | **Gew.-%** |
|---|---|
| B₂O₃ | 5-20 |
| MeO₂(Me = Ti, Sn) | 20-40 |
| SiO₂ | 20-50 |
| Al₂O₃ | 2-7 |
| (Na/K)₂O | 5-15 |
| CaO | 0-3 |

Im weiteren wird beschrieben, wie entsprechend der Rezepturen der Tabellen 1 und 2 ein Dentalkeramik-Haftvermittler hergestellt wird, und es wird an konkreten Beispielen nachgewiesen, dass diese Erzeugnisse herausragende Hafteigenschaften aufweisen.

Im Beispiel 1 wird gemäß nachfolgender Tabelle 3 ein Haftvermittler mit relativ hohem Glasanteil hergestellt, wobei die Komponenten dieser Rezeptur bei Temperaturen von 750°C bis 1450°C geschmolzen und/oder gesintert und nach dem Abkühlen auf Korngrößen von 30 µm oder kleiner gemahlen werden. Die Materialien wurden dann dünn als wässriger oder alkoholischer Schlicker auf eine edelmetallfreie Basislegierung aufgetragen und bei den vom Legierungshersteller empfohlenen Temperaturen gebrannt.

**Tabelle 3**

| **Komponente** | **Gew.-%** | **Haftung (MPa) :** |
|---|---|---|
| B₂O₃ | 2, 5 | 81,3 |
| TiO₂ | 20 | |
| SiO₂ | 49,7 | |
| Al₂O₃ | 6,0 | Optischer Befund |
| K₂O | 11, 1 | negativ |
| Na₂O | 7,3 | |
| CaO | 3,3 | |

Die Prüfung der Haftkraft erfolgte gemäß der Normvorschrift (EN ISO 9693:2000). Normgerecht dimensionierte Stäbe einer Legierung, z. B. "Remanium 2001" der Firma Dentaurum, wurden in einer dünnen Schicht (< 20 µm der mit Wasser angerührten Materialien über eine Länge von 8 mm beschichtet und bei einer Temperatur von 950-990°C gebrannt. Danach wurde der gebrannte Haftvermittler mit Initial PC-Pastenopaker der Firma GC zweifach verblendet, mit GC Initial MC-Dentin gebrannt und mit einem anschließenden Glanzbrand vollendet. Die Gesamtdicke der Beschichtung betrug entsprechend der Normvorgabe 1,1 mm.

Die Festigkeit des Metall-Keramik-Verbunds wurde entsprechend der Rissbeginnprüfung nach Schwickerath an einer Zwick-Universalprüfmaschine BZ010/TN2S untersucht. Die Bruchflächen wurden zusätzlich einer optischen Prüfung unterzogen, wobei das Vorhandensein von Keramikresten auf der Metallbruchfläche als positiver optischer Befund gewertet wurde.

Entsprechend zeigt sich, dass bei der Rezeptur gemäß Beispiel 1 eine überaus hohe Haftung von 81,3 MPa erreicht werden konnte, bei negativem optischem Befund.

Die nachfolgende Tabelle 4 variiert das Beispiel der Tabelle 3 hin zu einem Haftvermittler mit relativ geringem Glasanteil. Wiederum wurde in der oben beschriebenen Weise verfahren, wobei die rechte Spalte der Tabelle 4 das Ergebnis zeigt. Auch hier ist mit 79 MPa von sehr hoher Haftwirkung auszugehen; Keramikreste verbleiben auf der Metallbruchfläche.

**Tabelle 4**

| **Komponente** | **Gew.-%** | **Haftung (MPa):** |
|---|---|---|
| B₂O₃ | 29,8 | 79 |
| TiO₂ | 48, 5 | |
| SiO₂ | 13,9 | |
| Al₂O₃ | 1,7 | Optischer Befund |
| K₂O | 3,1 | positiv |
| Na₂O | 2,0 | |
| CaO | 0,9 | |

Schließlich zeigt als drittes Beispiel die Tabelle 5 einen Haftvermittler mit mittlerem Glasanteil, Verfahren wie oben. Hier ergeben sich praktisch vergleichbare Ergebnisse wie beim zweiten Beispiel der Tabelle 4.

**Tabelle 5**

| **Komponente** | **Gew.-%** | **Haftung (MPa):** |
|---|---|---|
| B₂O₃ | 10 | 79 |
| TiO₂ | 50 | |
| SiO₂ | 24, 8 | |
| Al₂O₃ | 3, 1 | Optischer Befund |
| K₂O | 5, 6 | positiv |
| Na₂O | 3,7 | |
| CaO | 1,7 | |

## Patentansprüche

1. Dentalkeramik-Haftvermittler zum Verbinden eines insbesondere mittels einer Nichtedelmetalllegierung realisierten Metall-Basisgerüsts (2) mit einer aufzubringenden dentalkeramischen Schicht (3,4,5),
**dadurch gekennzeichnet,**
**dass** der Haftvermittler (1) aus einem
- zwischen 10 und 50 Gew.-%, insbesondere zwischen 30 und 45 % Titan- und/oder Zinndioxid,
- zwischen 3 und 30 Gew.-% Bortrioxid
- und eine Glaskomponente
aufweisenden Haftvermittlermaterial hergestellt ist.

2. Haftvermittler nach Anspruch 1, **dadurch gekennzeichnet, dass** die Glaskomponente zwischen 5 und 20 Gew.-% Al₂O₃ enthält.

3. Haftvermittler nach Anspruch 1, **dadurch gekennzeichnet, dass** die Glaskomponente K₂O und/oder Na₂O im Bereich zwischen jeweils 0 und 20 Gew.-% enthält.

4. Haftvermittler nach einem der Ansprüche 1 bis 3, **dadurch gekennzeichnet, dass** die Glaskomponente CaO im Bereich zwischen 0 und 5 Gew.-% enthält.

5. Haftvermittler nach einem der Ansprüche 1 bis 4, **dadurch gekennzeichnet, dass** die Glaskomponente B₂O₃ im Bereich zwischen 0 und 30 Gew.-% enthält.

6. Haftvermittler nach einem der Ansprüche 1 bis 5, **dadurch gekennzeichnet, dass** das Haftvermittlermaterial Fe₂O₃ und/oder V₂O₅ im Bereich zwischen 0 und 3 Gew. -% enthält.

7. Verwendung des Haftvermittlers nach einem der Ansprüche 1 bis 6 zur Herstellung einer Zahnrestauration, bestehend auf einem vorzugsweise mit einer Nichtedelmetalllegierung realisierten Metall-Basisgerüst (2), der erfindungsgemäßen Verbindungsschicht (1) sowie der Opakerschicht einer Verblenkkeramik (3).

8. Verwendung nach Anspruch 7, **dadurch gekennzeichnet, dass** die Verbindungsschicht eine Dicke zwischen 5 und 20 µm aufweist.

9. Verfahren zum Herstellen einer Zahnprothese, **gekennzeichnet durch** die Schritte:
- Herstellen eines wässrigen oder alkoholischen Schlickers aus dem Haftvermittler-Material nach einem der Ansprüche 1 bis 6,
- Aufbringen des Schlickers auf ein insbesondere mittels einer Nichtedelmetalllegierung realisiertes Metall-Basisgerüst,
- Brennen des aufgebrachten Schlickers bei einer vorbestimmten Brenntemperatur und
- Aufbringen mindestens einer weiteren dentalkeramischen Schicht, insbesondere Opakerschicht, auf die gebrannte Beschichtung.

10. Verfahren nach Anspruch 9, **dadurch gekennzeichnet, dass** die Brenntemperatur auf einen Bereich zwischen 950°C und 1000°C eingestellt wird.

11. Verfahren nach Anspruch 9 oder 10, **dadurch gekennzeichnet, dass** der Schlicker durch Schmelzen und/oder Sintern des Haftvermittler-Materials nach einem der Ansprüche 1 bis 6 sowie dem Mahlen auf Korngrößen < = 30 µm nach dem Abkühlen hergestellt wird.
